# EUROPEAN PATENT SPECIFICATION

(11) **EP 4 370 899 B1**
(45) Date of publication and mention of the grant of the patent: **03.12.2025**
(21) Application number: 22743881.9
(22) Date of filing: 18.07.2022
(51) Int. Cl.: G01N 15/02, G01N 15/14, G01N 33/557, G03H 1/00, G01N 15/00, G01N 15/10

(54) **METHOD OF DETERMINING THE IDENTITY OF A BIOMOLECULE**
METHODE ZUR BESTIMMUNG DER IDENTITÄT EINES BIOMOLEKÜLS
PROCÉDÉ DE DÉTERMINATION DE L'IDENTITÉ D'UNE BIOMOLECULE

(30) Priority: 16.07.2021 GB 202110264
(43) Date of publication of application: 22.05.2024
(73) Proprietor: Refeyn Ltd, Oxford Business Park Oxford OX4 2HN (GB)
(72) Inventor: LANGHORST, Matthias, Oxford OX4 2HN (GB); BISHOP, Joshua, Oxford OX4 2HN (GB)
(74) Representative: Stratagem IPM Limited
(86) International application number: PCT/GB2022/051853
(87) International publication number: WO 2023/285842

(56) References cited:
- WO-A2-2020/104814
- US-A1- 2018 275 097
- US-A1- 2020 096 472
- GOLDFAIN AARON M ET AL: "Dynamic Measurements of the Position, Orientation, and DNA Content of Individual Unlabeled Bacteriophages", JOURNAL OF PHYSICAL CEMISTRY, 11 April 2016 (2016-04-11), XP055904881, Retrieved from the Internet <URL:http://www.ncbi.nlm.nih.gov/pubmed/27063451> [retrieved on 20220324]

## Description

The present invention relates to an improved method for determining the identity of a biomolecule, notably distinguishing between variants of biomolecules, such as viral serotypes and the like. Such a method has potential to simplify the testing for biological manufacture and for diagnosing disease, monitoring environmental issues and determination of contaminants.

A biological medicine, or biologic may be described as a treatment containing complex active substances made by a biological process or derived from a biological source. "Biologics" is a broad term and encompasses a wide range of products such as cells, tissues, blood derived products, nucleic acids, recombinant proteins, antibodies, vaccines, gene therapy viruses, hormones, and enzymes. Biologic drugs are generally administered by injection or infusion and are already available to treat numerous diseases.

The production of many biologics includes the use of living cells such as human cells, animal cells or microorganisms, generally using genetic manipulation methods. For example, monoclonal antibodies may be produced in Chinese hamster ovary cells (CHO).

Biologics testing is a critical process during production, as these complex molecules may be altered by changes in their manufacturing processes; they may have sensitivity to factors that could cause aggregation and degradation which impacts biologic activity, product safety, and quality. Unintended or unexplained alterations in manufacturing processes can lead to differing product attributes (i.e., drift). Determining the exact nature of the product is therefore critical.

Quality, safety, and efficacy must be continually monitored and in order to meet strict regulatory requirements. For example, determining the presence, identity and concentration of contaminants during production is a regulatory requirement.

In biologics production, the exact nature of the product as well as identity and relative concentrations of contaminants may change over time due to increasing heterogeneity of the producing cells, the metabolic state of the cells as well as other process conditions in the bioreactor or in downstream purifications. Such drift is an analytical challenge as the multitude of possible changes (oligomerisation and protein assembly, post-translational modifications, changes in the purification efficiency or failure of processing steps) may lead to very different changes in the composition of the final product which can only analysed by combining a wide variety of analytical techniques probing various aspects of product quality. This results in a lengthy and expensive release testing procedure (Ramanan et al. BioDrugs 28, pages 363-372 (2014)). This is particularly important as biosimilars enter the market; these are biologics highly similar to another already approved biological medicine (the 'reference medicine'). Thus, it is possible that similar biologics will be produced by multiple manufacturers with different quality systems. Different patterns of product drift could result in clinically meaningful differences among biologics.

One class of biologics is monoclonal antibodies (MAbs), which have become one the most powerful therapeutic and diagnostic tools available. Recent advances in molecular biology, immunology, and development of robust production platforms will drive the development of more antibodies suitable to treat other diseases and conditions. It is expected that this will lead to development of larger industrial facilities to increase production to meet increasing demand. Those in this area are aware of the critical quality control attributes particular to antibodies, and scale-up considerations that need to be made. (Gaughan, C.L., Molecular Diversity volume 20, pages 255-270 (2016)).

Currently, drift and release or finished product testing may involve numerous testing protocols, including but not limited to: appearance, osmolality, pH, cell-based bioassays, endotoxin testing, various chromatography techniques: size exclusion chromatography; reversed-phase chromatography; ion-exchange chromatography, amino acid analysis, protein concentration determination, peptide mapping, glycan profiling, capillary gel electrophoresis, capillary isoelectric focussing, and host cell assays including sugar, vitamin, metabolite, protein (generally ELISA) and DNA determination.

There is a drive to provide more real-time release testing for biologics. Real-time release testing (RTRT) is defined as "the ability to evaluate and ensure the quality of in-process and/or final drug product based on process data, which typically includes a valid combination of measured material attributes and process controls" (Jiang et al., Biotech & Bioeng 2017, https://doi.org/10.1002/bit.26383). However, despite the potential improvements RTRT could provide, manufacturers are looking at the practicalities of implementing this approach, and therefore, is not yet fully benefitting from its promises. Many problems remain, including which instrumentation to use and when and where during manufacturing to perform assays.

One particular method of interferometric light scattering microscopy is mass photometry, which employs a particular spatial filter to enable higher sensitivity. Mass photometry provides an efficient and effective method to measure the mass and relative concentration of biomolecules in a sample. This technique measures the mass of individual biomolecules by capturing the light scattered by each molecule at an interface. Extraction of the scattering contrast provides an indirect measurement of the mass. Mass photometry builds on the principles of interference reflection microscopy and interferometric scattering microscopy. Through carefully controlled illumination, a novel spatial-filtering strategy in the detection beam path and careful image analysis, it has been demonstrated that the minute amount of light scattered by single molecules can be reliably detected and, more importantly, that it is directly correlated with molecular mass (Young et al., Science 2018, 360 (6387), 423-7). However, at present mass photometry and interferometric light scattering techniques in general cannot distinguish between particles of very similar molecular mass. Particles may have a very similar mass because they are variants of the same biomolecule, for example. Such variants could be differently glycosylated proteins. On occasions, the resolution is insufficient to distinguish between individual species. Thus, current methods of interferometric light scattering microscopy, in particular mass photometry cannot, in isolation, accelerate techniques such as release testing, as additional testing is required.

It is therefore an aim of the current invention to provide a method for distinguishing between biomolecules of similar mass. The biomolecules of similar mass may be related to each other, i.e. isoforms of the same biomolecule. The biomolecules of similar mass may be variants of the same biomolecule. It is additionally an aim of the current invention to provide an improved method to determine the identity of a biomolecule in a sample, such as to enable the discrimination between possible variants, for example isoforms, glycoforms, lipoforms, species, serotypes or mutants. Such determination would enable techniques such as release testing to be performed, wherein the sample is known to comprise a biomolecule. Thus, the method may be used to determine a particular characteristic of a molecule with a certain mass. In release testing, the predicted mass of the biomolecule may be known, and therefore determination of further characteristics of molecules with an appropriate mass may be determined.

Biological molecules play indispensable roles in biological processes, including disease development, so the accurate detection and/or identification of biomolecules is critical to disease diagnosis and therapy. Further, given that biological environmental and industrial samples and the like may also contain particles of a similar mass, a method to identify a biomolecule in a sample may have further applications.

Biological molecules are undoubtedly complex. Biological molecules produced in cells are processed according to the cellular pathways of the cell. Different processing even in a natural setting can result in the production of isoforms. Host cells may use different pathways for processing when compared to the originating cell for the biological molecule. This may results in differential processing, for example, changes in protein folding, changes in nucleic acid methylation, changes in glycosylation patterns, changes in surface charge and the like. The physical characteristics of the biological molecule can therefore be altered by processing in a different cell type. Biological molecules may be classified into four general classes, proteins, carbohydrates, lipids and nucleic acids, depending on the monomer unit employed. These monomer units vary and include different chemical groups, which can have differing attributes, for example amino acids (the building blocks of peptides and proteins) can be polar, non-polar, positively or negatively charged. Biological molecules may be composed of more than one kind of monomer, for example glycosylated proteins, glycosylated RNA, lipoproteins, lipid-modified oligonucleotides and glycolipids. In general, biomolecules are folded to some degree, placing some of the monomers in the core, and exposing other monomers at the surface. Biological molecules therefore have a multitude of chemical groups on their surface, all of which help to determine the physical characteristics of the biomolecule. Changes to the way the biological molecule is produced is likely to alter the surface chemistry, leading to a change in the characteristics, particularly of the surface. Similarly, if the molecule changes conformation and previously internal sections form part of the exterior surface, these will have particular characteristics too.

Any biomolecule, whether protein, carbohydrate, or nucleic acid, can be distinguished from other molecules based on differences in some physical characteristics. Physical characteristics include, but are not limited to size, defined as either length or mass, charge, binding affinities (such as the ability to form weak, noncovalent bonds i.e. hydrogen bonds, ionic bonds, and van der Waals attractions; plus favourable hydrophobic interactions) and hydrophobicity. Biomolecules therefore may be defined by their molecular weight (mass) and by their isoelectric point (pl), for example. The isoelectric or isoionic point of a biomolecule is the pH at which the biomolecule carries no net electrical charge and hence considered neutral. The pl of biomolecules is mainly dependent on the dissociation constant (Kd) of any ionisable groups. Arginine, aspartate, cysteine, histidine, glutamate, lysine, and glutamate are all exemplary ionisable groups in proteins and they play a major role in determining the pl of a protein. However, co-translational and post-translational modifications (including phosphorylation, methylation, alkylation) can have a significant role in determining the pl of a biomolecule. All biomolecules, including without limitation, viruses, nucleic acids, polysaccharides and lipids, will have an isoelectric point.

"Dynamic Measurements of the Position, Orientation, and DNA Content of Individual Unlabeled Bacteriophages", Journal of Physical Chemistry, 11 April 2016 (2016-04-11), by Aaron M. Goldfain et al is a non-patent literature document that is aimed at studying the position, orientation, and DNA content of unlabeled bacteriophages (phages) in solution near a planar, functionalised glass surface using holographic microscopy.

The present invention relies upon the interaction of a biomolecule with a surface. How a biomolecule interacts with a surface is determined by the properties or characteristics of that biomolecule. This can be exploited by selecting an appropriate surface to determine a particular property or characteristic, with or without altering one or more condition in which the biomolecule is contacted with the surface, for example temperature. The interaction of the biomolecule and the surface may be determined using interferometric light scattering microscopy. Such a technique interrogates the interface of the surface and the sample, and detects biomolecules that interact with the surface. Each biomolecule may be tracked by taking repeated measurements, giving a picture of the interaction of the biomolecule and the surface. This permits a determination of the mobility of the biomolecule on the surface.

The interaction between the surface and the biomolecule may be due to any kind of interaction, from hydrophobicity to covalent bond formation. The interaction between the surface and the biomolecule may be due to ionic/ electrostatic interactions. These interactions are due to the physical characteristics of the biomolecule.

The present invention thus provides a method of determining the identity of a biomolecule in a sample comprising determining the interaction of a biomolecule with a surface.

There is provided:
A method of identifying a biomolecule in a sample using an interferometric light scattering apparatus; said method comprising:
(i) contacting said sample with a first surface and taking a plurality of measurements of said biomolecule on said first surface and using the measurements to determine the mobility of said biomolecule on said surface;
(ii) repeating step (i) using a second surface and determining the mobility of said biomolecule on said surface;
(iii) using the mobility of the biomolecule determined in step (i) and step (ii) to construct a profile for said biomolecule;
(iv) comparing the profile obtained in step (iii) with at least one reference profile to identify the biomolecule.

The method of the present invention therefore comprises several steps which may be performed in any appropriate order.

A surface may be selected such that the surface chemistry permits determination of at least one physical characteristic of the biomolecule. The surface may be selected to determine the interaction of the biomolecule with the surface, most notably the mobility of the biomolecule on the surface.

The surface is contacted with the sample, which may contain the biomolecule. The condition or conditions under which the surface is contacted with the sample may be selected to permit determination of at least one physical characteristic of the biomolecule. The surface may be selected to determine the interaction of the biomolecule with the surface, most notably the mobility of the biomolecule on the surface.

The identity of a biomolecule in a sample may be determined by determining the mobility of the biomolecule on the surface by creating or preparing a profile, which profile includes the data obtained from at least a first surface and a second surface (for example steps (i) and (ii)).

The first and second surface may be different surfaces.

The first and second surfaces may be the same surface. In this scenario, the second surface is contacted with the sample under at least one altered condition (when compared to the first surface). An altered condition may be selected from any one of: temperature; pH; ionic strength, redox potential, electric potential applied between surface and solution and presence of a reagent. A reagent may be any reagent that alters the interaction of the biomolecule and the surface, such as any one or more of: detergent, chaotropic agent, reducing agent, buffer, ion, salt, solvent, denaturing agent, and/or cross-linking agent. A plurality of conditions may be altered as required.

Once the biomolecule is detected at the surface, repeated measurements may be taken of that biomolecule whilst it remains in contact with the surface. This enables tracking of the biomolecule as it moves across the surface, as appropriate. These repeated measurements are described as taking a "plurality of measurements" and may also be described as measurements taken over time or a period of time. The rate at which these measurements are taken may be selected appropriately depending on the expected mobility of the biomolecule. The rate may be in the range of 10Hz to 10kHz, optionally 50Hz to 5KHz, preferably 100Hz to 1kHz. Any suitable rate may be selected within or from these ranges. The plurality of measurements are thus taken of the biomolecule over time to monitor the mobility of the molecule on the surface.

Taking a plurality of measurements of the interaction of the biomolecule with the surface permits tracking of that biomolecule. Detection and/or tracking can be performed at the level of a single biomolecule.

Any measurement may be used to determine the mass of the biomolecule, optionally a measurement taken in step (i) or step (ii) may be used, or an independent measurement may be taken to determine mass.

A reference profile may be prepared by taking a known biomolecule and performing the method with at least a first and second surface. This reference profile may be single combined profile or a set of profiles obtained from each surface. A summary of the interactions (such as the mobility) of a biomolecule with a surface may be used to construct a profile. A profile may be defined as a set of characteristics that identify or are thought to identify a particular biomarker. The profile may be a summary or analysis of data, in any appropriate form, such as a graph or table, representing distinctive characteristics.

According to an alternative aspect of the invention, there is provided a method of identifying a biomolecule in a sample, said method comprising contacting said sample with a first surface and using an interferometric scattering apparatus to take a plurality of measurements of said biomolecule over time, and using the plurality of measurements to determine the mass and mobility of the biomolecule on the first surface to determine at least one physical characteristic of said biomolecule.

The following features may apply to any aspect of the invention:
The first surface may be selected such that the surface chemistry permits determination of at least one physical characteristic of the biomolecule.

The first surface is contacted with the sample, which may contain the biomolecule. The conditions under which the first surface is contacted with the sample may be selected to permit determination of at least one physical characteristic of the biomolecule.

The identity of a biomolecule in a sample may be determined by determining mass and mobility of the biomolecule on the first surface without further measurements being required. Alternatively, further measurements may be required, for example in more complex samples or where potential variants of the biomolecule have similar characteristics.

Further measurements may comprise:
contacting said sample with a second surface and using an interferometric scattering apparatus to take a plurality of measurements of said biomolecule over time, and using the plurality of measurements to determine the mass and mobility of the biomolecule on the second surface to determine at least one physical characteristic of said biomolecule.

Alternatively or additionally, further measurements may comprise:
contacting said sample with a first surface and altering one or more conditions of said sample to determine at least one physical characteristic of said biomolecule. Said conditions may be defined as the conditions that affect the binding of the biomolecule to the surface, thus affecting the reaction of the surface and the biomolecule.

The conditions of the sample that may be altered include, but are not limited to: temperature, pH, ionic strength, presence of chemical entities such as detergents, chaotropic agents, reducing agents, oxidising agents, redox reagents, buffers, ions, solvent, denaturing agent, and/or cross-linking agents.

If a further measurement is required, the measurements obtained with the different surface and/or altered conditions can be combined with or compared to the measurements obtained with the first surface to provide sufficient information to identify the biomolecule. This may result in the determination of the mass of the biomolecule and two or more physical characteristics.

Since multiple measurements on one or more surfaces or under one or more conditions may be applied, a profile consistent with a biomolecule may be determined. This profile may be sufficient alone to determine the identity of a biomolecule, or the profile may be compared to a reference profile previously obtained for a known biomolecule.

According to one aspect the present method provides a method of identifying a biomolecule in a sample, said method comprising:
contacting said sample with a first surface, and using an interferometric scattering apparatus to take a plurality of measurements of said biomolecule over time, and using the plurality of measurements to determine the mass and mobility of the biomolecule on the first surface, and using said mass and mobility to define a profile for the biomolecule which may be compared to a reference profile.

The method may be repeated with a second surface and/or with a second set of conditions in order to supplement the profile. The method may be repeated with a further surface and/or with a further set of conditions to supplement the profile.

According to one aspect, the present method provides a method of identifying a biomolecule in a sample, said method comprising:
contacting said sample with a first surface, and using an interferometric scattering apparatus to take a plurality of measurements of said biomolecule over time, and using the plurality of measurements to determine the mass and mobility of the biomolecule on the first surface,
contacting said sample with a second surface, and using an interferometric scattering apparatus to take a plurality of measurements of said biomolecule over time, and using the plurality of measurements to determine the mass and mobility of the biomolecule on the second surface,
and combining or comparing the measurements on the first and second surfaces to determine the identity of the biomolecule.

According to one aspect, the present method provides a method of identifying a biomolecule in a sample, said method comprising:
contacting said sample with a first surface under a first set of conditions, and using an interferometric scattering apparatus to take a plurality of measurements of said biomolecule over time, and using the plurality of measurements to determine the mass and mobility of the biomolecule on the first surface under the first conditions,
contacting said sample with a first surface under a second set of conditions, and using an interferometric scattering apparatus to take a plurality of measurements of said biomolecule over time, and using the plurality of measurements to determine the mass and mobility of the biomolecule on the first surface under the second conditions,
and combining or comparing the measurements under the first and second conditions to determine the identity of the biomolecule.

The following features may apply to any aspect of the invention:
The plurality of measurements over time may be used to determine the time of interaction of each biomolecule with the surface and/or the movements of a biomolecule over the surface. Such measurements may be considered to be the "mobility" of the biomolecule on the surface. Thus, the mobility may be defined as the distance travelled on the surface and/or the length of time the biomolecule interacts with the surface. Either or both may be measured. It is possible to measure the distance travelled by the biomolecule on the surface, it may be described as lateral or horizontal motion across the surface. Alternatively or additionally, the time of the interaction of the biomolecule with the surface may be measured. Such interaction time indicates motion from the surface, such that the biomolecule is no longer interacting with the surface but moves or diffuses into the sample solution. This form of mobility depends on the strength of the interaction of the biomolecule with the surface. The interaction between the surface and the biomolecule may be indicative of the characteristics of the biomolecule, thus providing information on the same.

### Figures

Fig. 1 is a schematic diagram of an interferometric light scattering (iSCAT) microscope;
Fig. 2 is an image captured by the iSCAT microscope;
Figure 3 depicts the distances moved by AAV5 and AAV9 in a sample, with the inset box showing the trajectories of motion of the virus particles on the surface;
Figure 4A depicts the distances moved by IgG molecules on glass; whilst Figure 4B depicts the distances moved by IgG molecules on trimethylchlorosilane modified surface. The decreased movement on the trimethylchlorosilane modified surface is depicted; and
Figure 5 is a depiction of a typical antibody molecule with possible groups available for interaction with the surface highlighted.

Because all biological molecules (biomolecules or biologics) have surfaces with exposed chemical entities, the characteristics of the biomolecule will depend upon those chemical entities. Exemplary entities will include amino acid side chains in proteins, nucleotide bases in single stranded nucleic acids, sugar-phosphate backbone in nucleic acids, the chemistry and arrangement of the monosaccharide units in glycosylated biomolecules, hydrophobic side chains and polar head groups of some lipids, and any interacting metal ions or solvents with any of such groups.

Simple perturbations in how these biomolecules are produced, including in disease states or in transgenic animal or *ex vivo* manufacturing, will alter the surface characteristics of the biomolecule, for example via a change in folding of proteins, glycosylation or lipidation. It is an object of the invention to provide a method for determining the characteristics of a biomolecule. If the characteristics may be determined, then these may be compared to the "wild type" or expected characteristics, determined with a known sample of the biomolecule. This is particularly the case for manufacturing of biologics.

As used herein, the biomolecule may be described as an object, particle or species.

The present invention involves contacting a biomolecule with a surface. The biomolecule may interact with this surface, and it is this interaction that the present invention seeks to determine, based on the mobility of the biomolecule (on/off and/or lateral mobility). The particle is tracked and imaged over time, such that the time of the interaction of the particle with the surface can be determined and/or the distance moved along the surface. Such parameters are indicative of the strength of the interaction between the biomolecule and the surface, which is an indirect determination of the physical characteristics of the biomolecule. The physical characteristics are generally the characteristics of the external surface of the biomolecule, but the sample may be placed under conditions where internal portions of the biomolecule are exposed (denatured or unfolded).

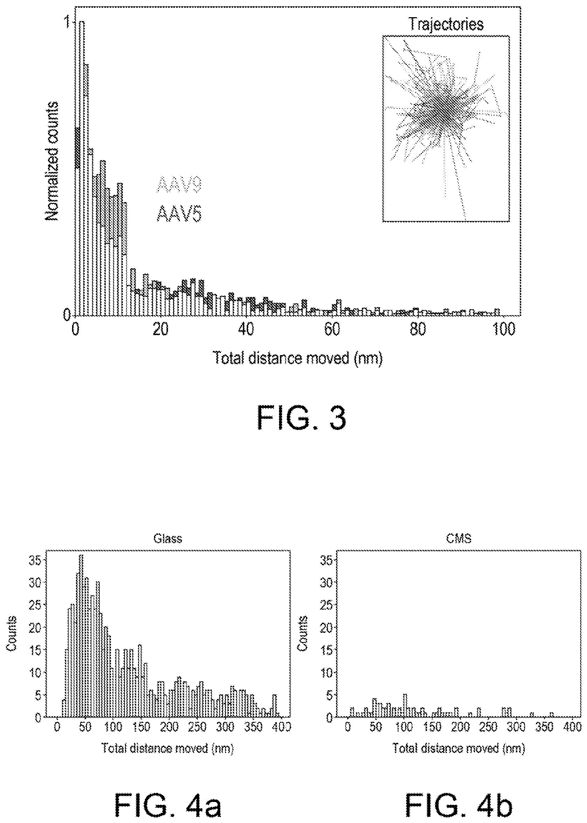

In order to determine characteristics of the biomolecule, the surface (first, second and/or further) may be selected to interact with particular chemical entities. Thus, the material from which the surface is composed may be selected on the basis of its ability to interact with the chemical entities present on or in the biomolecule. For example, the surface may be glass, and it is known that many biomolecules have a propensity to bind non-specifically to glass (for example, via protein adsorption). Figure 4A depicts the mobility of IgG on a glass surface, for example.

Proteins and lipids, for example, may be described amphiphilic macromolecules since they possess both hydrophobic and hydrophilic groups attracted to nonpolar and polar groups, respectively. Hydrophilic and charged groups tend to be located on the exterior surface of a particle. However, hydrophobic groups also exist on external surfaces too. Such groups may interact electrostatically with surfaces. Binding of the particle occurs when the free energy between the particle and the surface is less than that of the surface and water (or other solvent).

A surface for use in the present invention may be any suitable surface which is compatible with the interferometric detection method. A suitable surface may be capable of being functionalised or derivatised, or otherwise coated to alter the surface chemistry.

A surface is suitably solid (i.e. not a gel or a liquid). Suitably the surface allows transmission of ultraviolet light (which may be defined herein as having wavelengths in the range from 10nm to 380nm); visible light (which may be defined herein as having wavelengths in the range from 380nm to 740nm); and/or infrared light (which may be defined herein as having wavelengths in the range from 740nm to 300µm). Suitably, a surface allows transmission of light in the visible light spectrum. Suitably, a surface is substantially transparent. A surface may be smooth or may be textured. A smooth surface is preferred. The surface may be patterned.

A surface may be of any suitable material, including for example but without limitation glass, diamond, plastic, polymeric material (e.g. cyclic olefin copolymer, polyvinyl, polyethylene (PE) including for example polyethylene terephthalate (PET) and high-density polyethylene (HDPE) and low-density polyethylene (LDPE), polyacrylate (acrylic), polystyrene (PS) including high impact polystyrene (HIPS), silicone, polyester (for example polylactic acid (PLA) or polylactic coglycolic acid (PGLA)), polyurethane, polypropylene (PP), polyamide (nylon), Acrylonitrile butadiene styrene (ABS), Polyethylene/Acrylonitrile Butadiene Styrene (PE/ABS), bakelite, rubber, latex, polycarbonate (PC), Polycarbonate/Acrylonitrile Butadiene Styrene (PC/ABS) and polyvinyl chloride including for example polyvinylidene chloride (PVDC),and sapphire.

A surface may include a lipid membrane, such as a lipid bilayer, lipid micelles, lipid nanodiscs and the like. The lipid bilayer may be any suitable bilayer, such as a supported lipid bilayer. The bilayer may be supported on a glass coverslip, for example. Such supported lipid bilayers as a surface for iSCAT are discussed in: Foley, E.D.B., et al. Mass photometry enables label-free tracking and mass measurement of single proteins on lipid bilayers. Nat Methods 18, 1247-1252 (2021) and Vala, M., Piliarik, M. Weighing single protein complexes on the go. Nat Methods 18, 1159-1160 (2021).

The lipid membrane may be provided in any appropriate format, with any suitable lipid and/or protein formulation as required. The lipid membrane may have any suitable geometry.

A surface may have any suitable geometry. For example, a surface may comprise a flat plate, such as a coverslip, or may be a well, plate, bead, channel, container, flow cell, flow chamber, microfluidic cell or chamber, or slide. A surface may be part of a larger geometry or device. A suitable surface may be a glass coverslip or a plastic coverslip, wherein the plastic is as described above in relation to the surface.

A surface preferably forms part of a sample holder. Said sample holder may be an element of a light scattering microscope. The sample holder may be a high surface-to-volume chamber.

A surface may be functionalised. A surface may be passivated, activated, coated, treated or derivatised. The surface may be a passivated surface. Passivation is the process of treating or coating a surface in order to enhance or reduce the chemical reactivity, thus increasing or decreasing the number of interaction events. Those skilled in the art will be aware of suitable passivating agents, examples of which include BSA, PVPA, DDS and/or PEG. Alternatively, the surface is not passivated.

A surface may be activated, coated, treated and/or derivatised to alter the chemistry of the surface. Functionalisation of a surface enables the determination of a particular characteristic of the biomolecule. Functionalisation of a surface may be to permit various interactions, such as chemical interactions (e.g. covalent bonding) or physical interactions (e.g. adsorption). If the surface is coated, derivatised or modified, thin surface alterations may be desirable. Altered surface layers that are too thick can change the light scattering properties of the surface. Alteration of only the outermost few molecular layers (3-10 nm) may be desirable.

Any suitable surface coating may be applied, in order to change the chemistry of the surface. The surface may be altered to increase hydrophobicity or increase hydrophilicity. Suitable methods and coatings will be known and available to persons skilled in the art, including for example and without limitation, functionalised PEG, silanes, carboxy-silanes, amines, aminosilane, trimethylchlorosilane, aldehyde modification with amine modification reagents such as APTES, epoxy modification using for example GOPTS, carboxylate modification using for example EDC, NHS, HOBt, TBTU, PAMAM; diazo using for example NaNO₂, and supramolecules for example calixarenes.

Suitably, changing the surface chemistry of the surface does not substantially affect the light transmission of the surface.

The nature, such as the chemistry, of the surface may have an impact on the biomolecule. For example, a hydrophobic coating may result in partial unfolding of proteins to expose a hydrophobic "internal" area, such that the determination of characteristics is further than the assessment of the external surface of the biomolecule. Alternatively, by offering different surface modifications, different binding characteristics may be tested, for example the introduction of different surface charges by coating amino or carboxyl-groups. At neutral pH, the amino group is protonated and the carboxyl group is deprotonated.

The biomolecule may interact with the surface in a number of different ways. The biomolecule may be absorbed onto the surface, which is favoured by hydrophobic interactions, and may therefore require a hydrophobic surface. The biomolecule may react chemically with the functional groups of the surface via free reactive groups such as amine or carboxyl groups. Such covalent bonds restrict movement/mobility, both laterally and on/off the surface. Hydrophobic, hydrogen, van der Waals, ionic and coordinate bonding types, along with electrostatic interactions may also occur between the biomolecule and the surface. These types of interaction may be particularly affected by the conditions, such that changes in temperature and pH may alter the interaction of the biomolecule with the surface.

The sample is contacted with the surface (first, second and/or further) and this may occur inside any apparatus for detecting light scattering. However, the step may be performed before the surface is placed into the apparatus.

If the sample is repeatedly tested using different surfaces and/or different conditions, it will be appreciated by those skilled in the art that it is preferred to use a fresh (previously unused) sample to contact each surface. Thus, the methods of the invention may be described as being performed on a portion or an aliquot of a sample, such that multiple portions or aliquots may be contacted with a plurality of surfaces in parallel.

The biomolecule may be any suitable biological molecule, including but not limited to proteins, polypeptides, peptides, glycoproteins, glycosaminoglycans, glycosphingolipids, lipoproteins, nucleic acids, oligomers, oligonucleotides, glycosylated nucleic acids, complexes of lipids and nucleic acids, lipids, glycolipids, triglycerides, phospholipids, steroids, carbohydrates, oligosaccharides and polysaccharides. As used herein, the biomolecule can be an oligomer of various units, and form a larger structure, for example a virus particle. A virus may be identified in precisely the same way as a less complex biomolecule, by assessing characteristics including mass and/or mobility on a surface. The external parts of a virion may be proteinaceous or involve a lipid membrane. The biomolecule may therefore be a virus or a virus-like particle (VLP). The biomolecule may be a lipoplex, a lipid shell or a lipid nanoparticle (LNP), such as an LNP designed to deliver pharmaceuticals, such as RNA therapeutics.

Biomolecules may be present in variant forms, for example isoforms, glycoforms, lipoforms, species, serotypes or mutants. It is an aim of several aspects of the invention to determine which variant biomolecule is present in the sample. For example, the methods of the present invention have been shown to be able to distinguish between virions of serotype AAV5 and AAV9, due to their differential movement across a surface. A profile based on this mobility could be used to distinguish between these AAV serotypes in an unknown sample.

Isoforms may be encoded by the same gene, but involve differential processing. The resulting biomolecule may have a similar function and a similar mass but different characteristics. A serotype or serovar is a distinct variation within a species of virus. A glycoform is an isoform of a biomolecule that differs only with respect to the number or type of attached glycan. Glycoproteins often consist of a number of different glycoforms, with alterations in the attached saccharide or oligosaccharide. Variations in glycosylation is particularly pertinent with immunoglobulins. Different isoforms of lipids may exist, termed lipoforms.

Since variants of a biomolecule generally have a similar mass, simply determining the mass may not provide sufficient information to more precisely determine the identity of the biomolecule. The present invention, therefore, seeks to determine more information about the characteristics of the biomolecule, for example to determine charge, pl, or presence of particular chemical groups, such that the variant may be more closely identified. These could be described as the structural and physio-chemical characteristics of the biomolecule. For example, in the case of monoclonal antibodies important characteristics include mass, N/C-terminal, charge, glycosylation pattern, aggregation and FcgR binding. In manufacturing a biological molecule, the predicted mass of the product may be known, but the present method seeks to provide further information about the product with a certain mass. This method enables the determination of further characteristics of a biomolecule beyond a determination of mass. Such is particularly important where the process could result in the incorrect glycosylation of a protein product.

Proteins are composed of amino acid monomers, and these provide a diverse range of side chains which provide for many protein characteristics. Amino acid side chains vary in terms of size, charge, shape, hydrogen-bonding capacity, hydrophobic character, and chemical reactivity.

To enable this, the biomolecule is contacted with a surface, which may be prepared such that it provides different binding characteristics. Additionally or alternatively, the conditions can be altered to look for a specific effect. The conditions may be altered in any appropriate way, including but not limited to changing the pH, salt concentration, ionic strength, adding buffers, detergents, chaotropic agents and the like. Exemplarily, changing the pH may change the net charge on the biomolecule (due to protonation/deprotonation) and this will have an impact on charge-mediated surface binding. Altering salt concentrations can also be used to influence charge-mediated binding. Changing the ionic strength permits denaturation of double stranded nucleic acids for example. Detergents, denaturants, chaotropic agents can be used to partial unfold or dissociate biomolecules in order to examine non surface-exposed characteristics. Cross-linking agents can stabilise molecules to permit closer examination of surface chemistries. Alternatively or additionally, the redox potential may be altered, for example by altering the concentration of oxidants and/or reductants in the sample. Another example of an altered condition is the application of an electric potential between the surface and the solution, which would alter the attraction of the biomolecule to the surface.

The conditions of the sample that may be altered include, but are not limited to: temperature, pH, ionic strength, presence of chemical entities/reagents such as detergents, chaotropic agents, reducing agents, buffers, ions, solvent, denaturing agent, and/or cross-linking agents. Further conditions include the redox potential and the application of an electric potential between the surface and the sample.
The mass of the biomolecule may also be determined in the methods of the invention. The mass may be determined in any appropriate step, including any of steps (i) and/or (ii). An independent measurement step for determining the mass may be taken.

Interferometric light scattering accurately measures molecular mass by quantifying light scattering from individual biomolecules in solution. Such techniques have previously been described, such as in Young and Kukura, Annual Review of Physical Chemistry 2019 70:1, 301-322; Young et al, Science 27 Apr 2018: Vol. 360, Issue 6387, pp. 423-427 and Strack, R. Weighing single proteins with light. Nat Methods 15, 477 (2018).

The sample may be any suitable sample, but is preferable taken from biomolecule manufacturing, such that a known biomolecule is present, and the method is used to determine whether the correct variant is present, and/or the presence of unwanted contaminants. The sample is therefore preferably not a complex sample with a plurality of different unknown biomolecules present, since the presence of many biomolecules of a similar mass may be confounding. The sample is preferably liquid, such as a solution or suspension.

The sample may be a biological sample, such as a medical or veterinary sample. Such biological sample may be a bodily fluid of any appropriate type, or may be a suspension of a tissue of any appropriate type. The sample may be an environmental sample. Such environmental sample may be a water sample, or a sample taken from an environment and suspended in a solvent.

If the sample is suspected of being concentrated, it may be appropriately diluted prior to contacting the sample with a surface. Dilution may use any appropriate solvent, such as water. In Examples 1 and 2 the sample is appropriate diluted to ensure the number of biomolecule/surface interaction events measured are within a suitable level for the device to permit detection and tracking.

In the methods described herein, any suitable apparatus may be used. Standard iScat apparatus or Interference Reflection Microscopes may be suitable for some embodiments. Mass photometers available from Refeyn, UK may be suitable. In general, no labelling of the biomolecule is required. In general, the biomolecule is not tethered to the surface.

The present invention involves the use of interferometric light scattering apparatus, such as a microscope or mass photometer. Such devices interrogate the interface between the sample and the surface. Such an apparatus may take a plurality of measurements. The biomolecules on the surface scatter light, producing contrast in the measurement. It is possible to localise each scattering event (and thus each biomolecule) by fitting the contrast signal to a profile (such as a Gaussian profile) and localising that event to the peak of that fit. Repeated measurements may be taken at a suitable rate, depending on the expected speed of the movement of the biomolecule. A suitable rate at which the measurements are taken is the range of 10Hz to 10kHz, optionally 50Hz to 5KHz, preferably 100Hz to 1kHz. Any suitable rate may be selected within or from these ranges. Individual biomolecules may thus be tracked by using repeated fitting of the contrast signal and repeated localisation, creating a "trajectory" of the biomolecule over the surface. If the contrast signal disappears, this is indicative that the biomolecule has detached from the surface. Measurements that may therefore be taken to indicate mobility include, but are not limited to: time associated with the surface, distance travelled on the surface, speed of movement on the surface and/or frequency of interaction with the surface (measurement of association/dissociation with surface).

The measurements taken may be used to construct a profile for the biomolecule. For example, normalised counts for distance moved can be plotted, or the speed the biomolecule moves can be plotted. Alternatively, the trajectory of the biomolecule can be plotted (see Figure 3 inset); the trajectory being the path taken by the biomolecule across the surface. The constructed profile may therefore reflect various characteristics or properties of the biomolecule, according to their interaction with the surface.

A reference profile may be prepared by taking a known biomolecule and performing the method as described with at least a first and second surface. This reference profile may be single combined profile based on multiple data sets for two or more surfaces, or may be a set of profiles obtained from each surface individually. A summary of the interactions (such as the mobility) of a biomolecule with a surface may be used to construct a profile. A profile may be defined as a set of characteristics that identify or are thought to identify a particular biomarker. The profile may be a summary or analysis of data, in any appropriate form, such as a graph or table, representing distinctive characteristics. The reference profile may be stored in a database permitting comparison to take place by computer-implemented means. Figures 3 and 4 contain representations of the collected data that may be used to construct a profile for a biomarker.

The profile constructed using the methods of the invention (for example, using steps (i) to (iii) of the method) may be compared to a reference profile. This entails examining the biomarker profile and a reference profile to find similarities. Similarities in profile attributes enables identification of the biomolecule. For an unknown sample to be compared to a reference sample, it will be appreciated by those skilled in the art that the comparison has to be meaningful, such that the same characteristics are determined for both the reference biomarker and the biomarker in the sample. Generally, this will mean that the first and second (and any further) surfaces are the same for both the reference biomarker and the sample biomarker. However, there may be some instances wherein one or more surfaces are interchangeable when it comes to determining a characteristic, and thus the surfaces should be of the same class for the reference and sample profiles. For example a surface that can be used to determine hydrophobicity could have exposed methyl (- CH₃), methylene (- CH₂-), methoxy (- OCH₃), or trifluoromethyl ester (- OCF₃) groups, or alternatively comprise or be coated with polymeric biomaterials for example, PTFE, PDMS, PET, polyethylene, and polypropylene. Thus, a surface may be selected from a class of surfaces designed to investigate a particular property or characteristic of a biomolecule. A comparison step may therefore involve comparing an unknown biomarker profile on a "hydrophobic" surface and comparing it to a reference biomarker profile which entailed the use of an alternative "hydrophobic" surface.

In the apparatus, microscopes and methods described herein, the light used may be: ultraviolet light (which may be defined herein as having wavelengths in the range from 10nm to 380nm); visible light (which may be defined herein as having wavelengths in the range from 380nm to 740nm); infrared light (which may be defined herein as having wavelengths in the range from 740nm to 300µm). The light may be a mixture of wavelengths. Herein, the terms 'optical' and 'optics' are used to refer generally to the light to which the methods are applied.

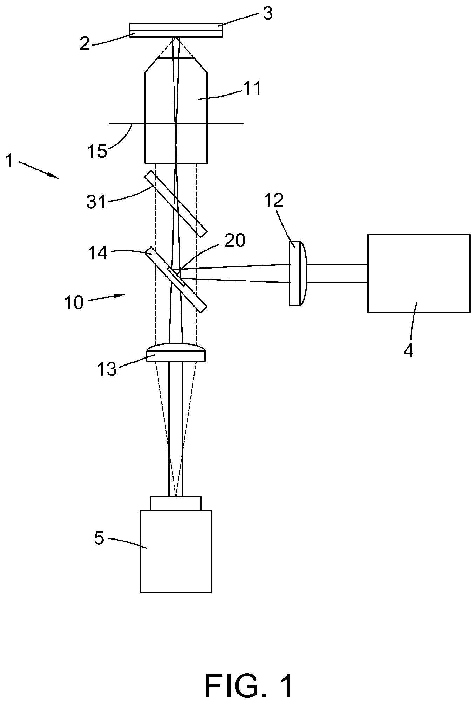

Fig. 1 illustrates an iSCAT microscope 1 which is arranged as follows.

The microscope 1 includes the following components that, except for the spatial filter described in more detail below, have a construction that is conventional in the field of microscopy.

The microscope 1 comprises a sample holder 2 for holding a sample 3 at a sample location. The sample 3 may be a liquid sample comprising objects to be imaged, which are described in more detail below. The sample holder 2 may take any form suitable for holding the sample 3. Typically, the sample holder 2 holds the sample 3 on a surface, which forms an interface between the sample holder 2 and the sample 3. For example, the sample holder 2 may be a coverslip and/or may be made from glass. The sample 3 may be provided on the sample holder 2 in a straightforward manner, for example using a micropipette.

The microscope 1 further comprises an illumination source 4 and a detector 5.

The illumination source 4 is arranged to provide illuminating light. The illuminating light may be coherent light. For example, the illumination source 4 may be a laser. The wavelength of the illuminating light may be selected in dependence on the nature of the sample 3 and/or the properties to be examined. In one example, the illuminating light has a wavelength of 405nm.

Optionally, the illumination light may be modulated spatially, to remove speckle patterns that arise from the coherent nature of the illumination and laser noise, for example as detailed in Kukura et al., "High-speed nanoscopic tracking of the position and orientation of a single virus", Nature Methods 2009 6:923-935.

The detector 5 receives output light in reflection from the sample location. Typically, the microscope 1 may operate in a wide-field mode, in which case the detector 5 may be an image sensor that captures an image of the sample 3. The microscope 1 may alternatively operate in a confocal mode, in which case the detector 5 may be an image sensor or may be a point-like detector, such as a photo-diode, in which case a scanning arrangement may be used to scan a region of the sample 3 to build up an image. Examples of image sensors that may be employed as the detector 5 include a CMOS (complementary metal-oxide semiconductor) image sensor or a CCD (charge-coupled device).

The microscope 1 further comprises an optical system 10 arranged between the sample holder 2, the illumination source 4 and the detector 5. The optical system 10 is arranged as follows to direct illuminating light onto the sample location for illuminating the sample 3, and to collect output light in reflection from the sample location and to direct the output light to the detector 5.

The optical system 10 includes an objective lens 11 which is a lens system disposed in front of the sample holder 2. The optical system 10 also includes a condenser lens 12 and a tube lens 13.

The condenser lens 12 condenses illuminating light from the light source 11 (shown by continuous lines in Fig. 1) through the objective lens 11 onto the sample 3 at the sample location.

The objective lens 11 collects the output light which comprises both (a) illuminating light reflected from the sample location (shown by continuous lines in Fig. 1), and (b) light scattered from the sample 3 at the sample location (shown by dotted lines in Fig. 1). The reflected light is predominantly reflected from the interface between the sample holder 2 and the sample 3. Typically, this is a relatively weak reflection, for example a glass-water reflection. For example, the intensity of the reflected illuminating light may be of the order of 0.5% of the intensity of the incident illuminating light. The scattered light is scattered by objects in the sample 3.

In a similar manner to conventional iSCAT, scattered light from objects at or close to the surface of the sample constructively interfere with the reflected light and so are visible in the image captured by the detector 5. This effect differs from a microscope operating in transmission wherein the illuminating light that reaches the detector is transmitted through the depth of the sample leading to a much smaller imaging contrast.

As shown in Fig. 1, the reflected illuminating light and the scattered light have different directionalities. In particular, the reflected illuminating light has a numerical aperture resulting from the geometry of the beam of light output by the light source 4 and the optical system 6. The scattered light is scattered over a large range of angles and so fills larger numerical aperture than the reflected illuminating light.

The tube lens 13 focuses the output light from the objective lens 11 onto the detector 5.

The optical system 6 also includes a beam splitter 14 that is arranged to split the optical paths for the illuminating light from the light source 4 and the output light directed to the detector 5. Except for the provision of a spatial filter as described below, the beam splitter 14 may have a conventional construction that provides partial reflection and partial transmission of light incident thereon. For example, the beam splitter 14 may be a plate, typically provided with a film, which may be metallic or dielectric, arranged at 45° to the optical paths. Alternatively, the beam splitter 14 may be a cube beam splitter formed by a matched pair of prisms having a partially reflective film at the interface between the prisms. Alternatively, the beam splitter 14 may be a polarising beam splitter, used in combination with a quarter wave plate between the beam splitter 14 and the sample 3.

In the example shown in Fig. 1, the light source 4 is offset from the optical path of the objective lens 11 so that the illuminating light from the light source 4 is reflected by the beam splitter 14 into the objective lens 11, and conversely the detector 5 is aligned with the optical path of the objective lens 11 so that the output light from the sample location is transmitted through the beam splitter 14 towards the detector 5.

In addition to the components described above that may be of a conventional construction, the microscope 1 includes a spatial filter 20. In the example shown in Fig. 1, the spatial filter 20 is formed on the beam splitter 14 and is thereby positioned behind the back aperture of the objective lens 11, and so directly behind the back focal plane 15 of the objective lens 11. Thus, the spatial filter 20 may be implemented without entering the objective lens as in phase contrast microscopy. Placing the spatial filter directly behind the entrance aperture of the objective rather than in a conjugate plane (for example as described below) has the distinct advantage of strongly suppressing back reflections originating from the numerous lenses within high numerical aperture microscope objectives. This, in turn, reduces imaging noise, lowers non-interferometric background and reduces the experimental complexity, number of optics and optical pathlength leading to increased stability of the optical setup and thus image quality.

However, this location is not essential and a spatial filter having an equivalent function may be provided elsewhere as described below.

The spatial filter 20 is thereby positioned to filter the output light passing to the detector 5. In the example shown in Fig. 1 in which the detector 5 is aligned with the optical path of the objective lens 11, the spatial filter 20 is therefore transmissive.

The spatial filter 20 is partially transmissive and therefore passes the output light, which includes the reflected illumination light, but with a reduction in intensity. The spatial filter 20 is also aligned with the optical axis and has a predetermined aperture so that it provides a reduction in intensity within a predetermined numerical aperture. Herein, numerical aperture is defined in its normal manner as being a dimensionless quantity characterising a range of angles with respect to the sample location from which the output light originates. Specifically, the numerical aperture NA may be defined by the equation NA=n•sin(θ), where θ is the half angle of collection and n is the refractive index of the material through which the output light passes (for example the material of the components of the optical system 6).

The spatial filter 20 provides no intensity reduction outside the predetermined numerical aperture. In principle, the spatial filter 20 could alternatively provide a reduction in intensity outside its predetermined aperture, but a reduction in intensity that is less than the reduction in intensity within the predetermined numerical aperture, although this is less desirable.

The spatial filter 20 may be formed in any suitable manner, typically comprising a layer of deposited material. The material may be, for example, a metal such as silver. The deposition may be performed using any suitable technique.

As sub-diffraction sized objects near an interface scatter light preferentially into a larger numerical aperture than the reflected illuminating light, the reduction in intensity provided by the spatial filter 20 preferentially reduces the intensity in detection of the reflected illuminating light over the scattered light. Accordingly, the reduction in intensity by the spatial filter 20 at low numerical apertures predominantly affects the reflected illuminating light and has a minimal effect on the scattered light, thereby maximising the contrast in the capture image. The enhanced imaging contrast enables high contrast detection of objects that are weak scatterers.

The contrast enhancement may be understood as follows. As the spatial filter 20 passes part of the output light in the predetermined numerical aperture (i.e. is partially transmissive in this example), fractions of illuminating light and scattered light fields reach the detector and interfere for a sufficiently coherent illumination source. The light intensity reaching the detector I_{det} is then given by I_{det} = |*E_{inc}*|*²*{*r²t²+*|*s*|²+2*rt*|*s*|cos*Φ*}, where *E_{inc}* is the incident light field, *r*² is the reflectivity of the interface and *t*² is the transmissivity of the spatial filter 20, *s* is the scattering amplitude of the object, and *Φ* is the phase difference between transmitted illuminating light and the scattered light. Thus, the scattering contrast is enhanced, albeit at the expense of the total number of detected photons.

Thus, contrast is provided in a similar manner to conventional iSCAT, but controlled additionally by the transmissivity of the spatial filter. This provides the ability to tune the amplitude of the reference field directly through selection of the transmissivity *t*² of the spatial filter 20 as opposed to being fixed by the reflectivity of a glass-water interface as in standard iSCAT. In the case that the spatial filter 20 is a layer of deposited material, the transmissivity *t*² may be selected by choice of the material and/or thickness of the layer. Such tuning may be performed according to, for example, the scattering object of interest, the camera full well capacity and magnification.

To maximise these beneficial effects to iSCAT, the predetermined numerical aperture may be the numerical aperture of the reflected illuminating light within the output light, but that is not essential. For example, benefits of a similar nature could be achieved if the predetermined numerical aperture was slightly smaller than, or larger than the numerical aperture of the reflected illuminating light.

Use of the spatial filter 20 does not fundamentally alter the sensitivity limits or SNR (signal to noise ratio) achievable for scattering by a given object, incident light intensity and exposure time. However, by improving the contrast and reducing the overall detected photon flux, it does, however, dramatically simplify the implementation of iSCAT to achieve a given sensitivity or SNR. Existing iSCAT microscopes have complex and expensive components, for example requiring an optical table, and expensive and complex optics, electronics, as well as needing expert operation. Such requirements are greatly relaxed by the use of the spatial filter 20. Equivalent performance to existing iSCAT microscopes may be achieved, for example, simply by adding the spatial filter 20 to an existing commercial microscope that does not have the complex and expensive components mentioned above. The spatial filter 20 itself is of course a simple and cheap component. In addition, the spatial filter 20 enables use of standard CMOS or CCD cameras with low full well capacity without loss of imaging sensitivity.

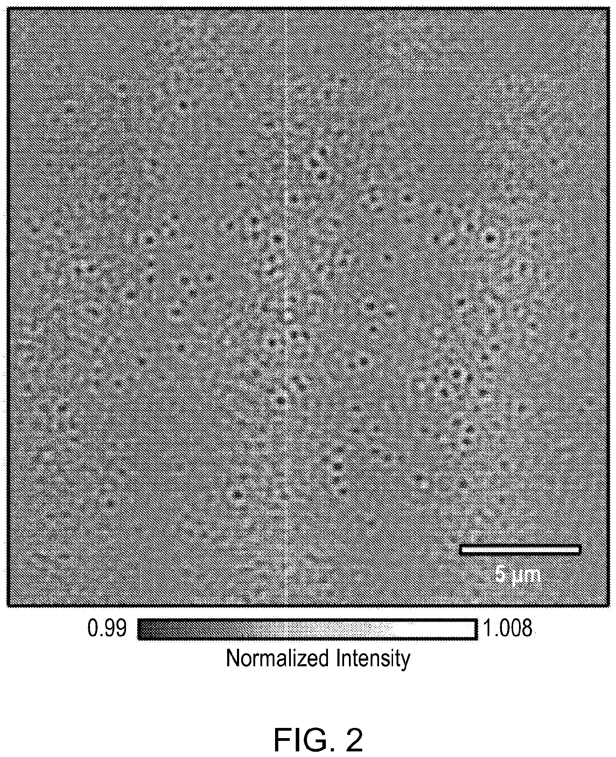

An image acquired using an example of the microscope 1 is shown in Fig. 2. In this example, coherent brightfield illuminating light was provided and the spatial filter 20 comprised by a layer of silver of thickness 180 nm deposited on fused silica with a 3.5 mm diameter so as to transmit 1×10⁻² of the reflected light intensity. This results in a scattering contrast of 1% for a single 395 kDa protein and a SNR of 10 (at an image capture rate of 10 frames s⁻¹, and with an intensity of illuminating light of 10 kW/cm²). Fig. 2 is an image captured using a low cost CMOS camera as the detector 5. As can be seen, a high contrast image is achieved. Moreover, brightfield illumination ensures that the strongest unwanted back-reflections, usually originating from the objective are directed away from the detector 5, minimising imaging background and enabling large fields of view without complex scanning of the beam of illuminating light.

The advantages of enhanced contrast allow imaging of objects that scatter light so weakly that imaging with other techniques is difficult. For example, the present invention may be applied with advantage to a sample comprising objects having a mass of 5000 kDa or less. Typically, the present invention may be applied to a sample comprising objects having a mass of 10 kDa or more, for example objects having a mass within a range from 10 kDa to 5000 kDa.

Alternatively or as well, the present invention may be applied to a sample comprising objects having a scattering cross section with respect to the illuminating light of 10⁻¹² m² or less, or more preferably 10⁻¹⁷ m² or less. Typically such objects may also have a scattering cross section with respect to the illuminating light. Typically such objects may also have a scattering cross section with respect to the illuminating light of 10⁻²⁰ m², or more preferably 10⁻²⁶ m² or more, for example within a range from 10¹⁷ m² from 10⁻²¹ m². Scattering cross section is a fundamental, measurable property relating to the effective size of an object to incident light of a particular wavelength, independent of the technique used to measure it. Scattering cross sections can be, for example, measured by dark field microscopy. Examples of objects to which the present invention may be applied include proteins or small aggregates thereof, or their binding partners.

In order to image objects that are relatively weak scatterers, the spatial filter 20 may be arranged to pass reflected illuminating light with a reduction in intensity within the predetermined numerical aperture to an intensity in the range from 10⁻² to 10⁻⁴ of the incident intensity (in this context, the intensity of the output light that is incident on the spatial filter 20).

Otherwise, the microscope 1 may be designed and operated without reference to the spatial filter 20. For example, the field of view is adjustable by changing the focusing conditions of the illumination light. Similarly, multi-colour imaging requires no more than coupling additional laser sources into a single mode fibre, if such a fibre is used to deliver the illumination light. In general terms, the microscope 1 may be adapted to use other components and techniques known for iSCAT, for example as disclosed in Kukura et al., "High-speed nanoscopic tracking of the position and orientation of a single virus", Nature Methods 2009 6:923-935, and in Ortega-Arroyo et al. "Interferometric scattering microscopy (iSCAT): new frontiers in ultrafast and ultrasensitive optical microscopy", Physical Chemistry Chemical Physics 2012 14:15625-15636.

### First Method

A sample suspected or known to comprise a biomolecule is placed on a sample holder with a glass surface. The microscope 1 takes a plurality of measurements of the particles in the sample at the surface at a rate of 100 Hz to 1 kHz. Individual particles contacting the surface scatter the measurement light, producing contrast in the measurement. Each scattering event is localised by fitting the contrast signature to a profile (e.g. a Gaussian profile) and localizing the event to the peak of that fit. Individual particles are tracked using repeated fitting of the contrast signal and repeated localisation creating trajectories of each particle over the surface. This enables the "lateral" mobility to be monitored and thus measured. However, if the contrast signal disappears, the particle has detached from the surface, and this is indicative of mobility on/off the surface (indicating particular characteristics in terms of interaction with the surface). From these measurements, the distance each biomolecule moves during its interaction with the surface can be calculated, or the length of time the biomolecule interacts with the surface can be measures to determine the mobility, and the molecular mass of each biomolecule may be calculated. Normalised counts for the distance moved can be plotted, as shown in Figure 3. How far a biomolecule moves during interaction with the surface indicates the strength of interaction with the surface and can therefore indicate different characteristics about the biomolecule. How often the biomolecule moves away from the surface and/or how long the biomolecule interacts with the surface also indicates the strength of the interaction with the surface and can indicate different characteristics about the biomolecule. Either or both measurements note the mobility of the molecule. As it can be seen, the two samples used in Figure 3 each contained biomolecules - Adeno-Associated Viruses (AAVs): AAV5 and AAV9 virus particles. It can be seen that AAV5 and AAV9 have different mobilities on the surface, with AAV9 having a more pronounced range of mobility between 0-10nm range. Different AAV serotypes have variations on the surface of their capsids that may be involved in receptor binding, host tropism, and antigenic determinants. Thus, the method can make a determination between AAV5 and AAV9 based on their different capsid chemistries, since this alters their mobility on the surface. Thus, this data can be used to construct a reference profile for AAV5 and/or AAV9 which can be used to determine the serotype of an unknown AAV sample by constructing a profile using the methods of the invention for the unknown sample.

The surface of the sample holder and/or the coating on the surface of the sample holder can be chosen to determine specific characteristics of the biomolecule, either the characteristics of the external surface of the biomolecule, or internal characteristics can be determined, i.e. by exposing a protein to a hydrophobic surface which can cause unfolding, or by adding a denaturant to the sample to purposively unfold the biomolecule.

The method described above uses the distance moved as indicative of the mobility. However, the time of interaction could equally be used or, alternatively, a combination of the time of interaction and the distance moved - for example, the speed of movement.

### Second Method

A sample comprising an immunoglobulin IgG is placed on a sample holder comprising a surface, which is either glass or a glass surface coated with TMCS (trimethylchlorosilane modified surface) and the mobility is measured (as for the first method). The microscope 1 takes a plurality of measurements of the particles in the sample at the surface at a rate of 100 Hz to 1 kHz. From these measurements, the distance each biomolecule moves during its interaction with the surface can be calculated to determine the mobility, and the molecular mass of each biomolecule may be calculated. Normalised counts for the distance moved can be plotted, as shown in Figure 4a and 4b. Frequency, speed and distance of biomolecule movement during interaction with the surface indicate the strength of interaction with the surface and can therefore indicate different characteristics about the biomolecule. How often the biomolecule moves away from the surface and/or how long the biomolecule interacts with the surface also indicates the strength of the interaction with the surface and can indicate different characteristics about the biomolecule. Either or both measurements note the mobility of the molecule.

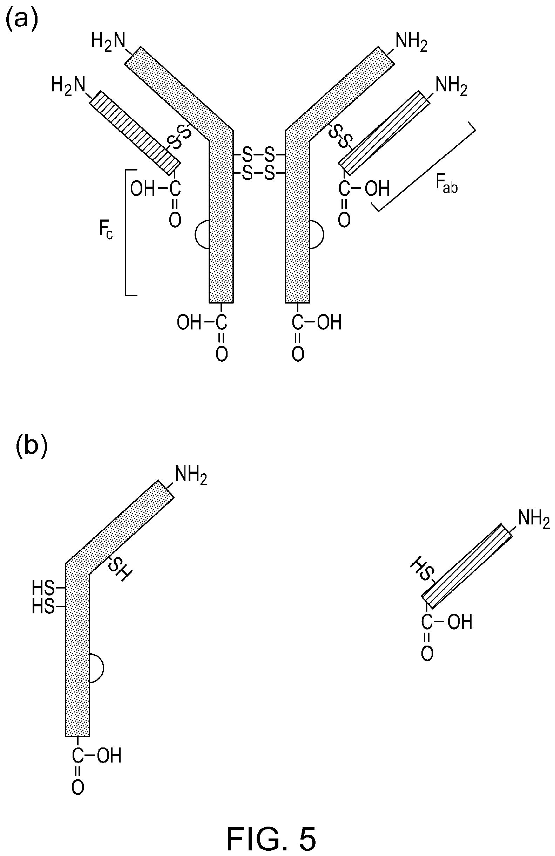

Immunoglobulin G (IgG) antibodies are large globular proteins with a molecular weight of about 150 kDa composed of two different kinds of polypeptide chain. One, of approximately 50 kDa, is termed the heavy chain, and the other, of 25 kDa, is termed the light chain. Each IgG molecule consists of two heavy chains and two light chains. The two heavy chains are linked to each other by disulphide bonds and each heavy chain is linked to a light chain by a disulphide bond. IgG may further be glycosylated. Figure 5 illustrates a typical IgG molecule, with two heavy (dotted bars) and two light (striped bars) chains. Each chain has carboxyl (-COOH) and amine (-NH2) groups, provided by terminal and internal amino acids. The heavy chains are linked with two disulphide bridges in the hinge region. Each light chain is linked to its respective heavy chain through a disulphide bridge. Amine and carboxyl groups are the reactive functional groups present in the physiological state. Sulfhydryl groups can be generated after reducing the disulphide bonds (such as by changing the conditions to include reducing reagents including DTT or TCEP). Carbohydrates can be activated by periodate treatment (oxidising agent). A net negative charge will be attained at a pH higher than its pl, which may be achieved using a buffer. Immunoglobulins may then be less mobile on positively charged surfaces since they are "immobilised" by ionic/ electrostatic interactions.

Silanes are silicon chemicals possessing a hydrolytically sensitive centre that can react with inorganic substrates (such as glass) to form stable covalent bonds and that possess an organic substitution which alters the physical interactions of treated substrates. Some of the properties of the surface that can be modified with silanes include: hydrophobicity, adhesion, release, dielectric, absorption, orientation, hydrophilicity, and/or charge conduction. Silane treatments of surfaces involve a liquid phase or vapour phase chemical reaction, and are straightforward to perform and of low cost. Silane reactions are most often used to modify hydroxylated surfaces. Since glass is rich in hydroxyl groups, silanes are particularly useful for modifying this material. Numerous silane compounds are commercially available, permitting a broad range of chemical functionalities to be incorporated on surfaces. The advantages of silane reactions are their simplicity and stability, attributed to their covalent, cross-linked structure.

As it can be seen, the IgG molecule is relatively mobile on glass, with some IgG molecules moving as far as 400nm whilst being monitored. However, in comparison on a modified surface, the amount of mobility is greatly restricted. This represents a different type of interaction between the IgG molecule and the surface. It is postulated that the IgG on the modified surface may form covalent bonds, which restricts mobility on the surface, and furthermore restricts the immunoglobulins from leaving the surface; or it is possible that the IgG has partially unfolded, restricting mobility. The mobility of this IgG on glass and a modified surface may be used to construct a reference profile for the IgG which can be used as a comparison when data on an unknown immunoglobulin is obtained.

Various further aspects and embodiments of the present invention will be apparent to those skilled in the art in view of the present disclosure. Unless context dictates otherwise, the descriptions and definitions of the features set out above are not limited to any particular aspect or embodiment of the invention and apply equally to all aspects and embodiments which are described.

It will further be appreciated by those skilled in the art that although the invention has been described by way of example with reference to several embodiments. It is not limited to the disclosed embodiments and that alternative embodiments could be constructed without departing from the scope of the invention as defined in the appended claims.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.
All mention of publications are herein incorporated by reference.
The invention will now be described with reference to several specific Examples, which are not limiting to the scope of the invention:

### Example 1

Adeno-associated viruses (AAVs) are non-pathogenic viruses composed of a protein shell surrounding and protecting a small, single-stranded DNA genome. The expected molecular weight (MW) of empty AAV capsids is about 3700 kDa. For all the examples described here, a mass photometer from Refeyn (Oxford, UK) was utilised.

A sample of known AAV5 or AAV9 was diluted to a final concentration of approximately 1x10¹¹ pp/ml or 1x10¹¹ vg/ml. The aim is to have 500 to 2000 events in the AAV mass range using the mass photometer. The sample was placed on a sample holder with a glass slide (in this instance a non-functionalised surface with mixed natural charge). The mass photometer takes a plurality of measurements of the AAV in the sample at the surface at a rate of 100 Hz to 1 kHz, generally 1 kHz. Individual AAV particles contacting the surface scatter the measurement light, producing contrast in the measurement. Each scattering event is localised by fitting the contrast signature to a profile (e.g. a Gaussian profile) and localising the event to the peak of that fit. Individual particles are tracked using repeated fitting of the contrast signal and repeated localisation creating trajectories of each AAV particle over the surface. This enables the "lateral" mobility to be monitored and thus measured. However, if the contrast signal disappears, the particle has detached from the surface, and this is indicative of mobility on/off the surface (indicating particular characteristics in terms of interaction with the surface). From these measurements the distance each AAV particle moves during its interaction with the surface can be calculated, or the length of time the AAV particle interacts with the surface can be measured to determine the mobility, and the molecular mass of each AAV particle may be calculated. From these measurements, the distance each AAV particle moves during its interaction with the surface was calculated to determine the mobility, and the molecular mass of each AAV was calculated (data not shown). Normalised counts for the distance moved was plotted, as shown in Figure 3. The experimental work was performed individually for AAV5 and AAV9, and the results plotted together to show the differences in mobility. Such data on the mobility on a glass surface may be used to construct a reference profile for AAV5 and AAV9, to permit a comparison when a sample containing an unknown serotype of AAV is investigated on the same surface. Thus, these AAV serotypes may be distinguished by their mobility on the surface, due to their differing capsid chemistries.

### Example 2

A sample of IgG (Sigma Aldrich, UK) was diluted such that 500 to 2000 events in the IgG mass range using the mass photometer was achievable and placed in a sample holder including either a glass surface or a sample holder including a surface derivatised with trimethylchlorosilane (Gelest, US). As in Example 1, the mass photometer takes a plurality of measurements of the IgG in the sample at the surface at a rate of 100 Hz to 1 kHz. From these measurements, the distance each IgG molecule moves during its interaction with the surface was calculated to determine the mobility, along with the molecular mass (data not shown). Normalised counts for the distance moved was plotted, as shown in Figures 4a and 4b. IgG has a substantially reduced mobility on the derivatised surface in comparison to glass, in line with a different mechanism of interaction with the derivatised surface. The mobilities of the IgG on the class and derivatised surfaces may be used to construct a reference profile for this IgG molecule. Such reference profile serves as a comparator for profiles from other immunoglobulins to determine the identity of the unknown immunoglobulin.

## Claims

1. A method of identifying a biomolecule in a sample using an interferometric light scattering apparatus; said method comprising:
(i) contacting said sample with a first surface and taking a plurality of measurements of said biomolecule on said first surface and using the measurements to determine the mobility of said biomolecule on said surface;
(ii) repeating step (i) using a second surface and determining the mobility of said biomolecule on said surface;
(iii) using the mobility of the biomolecule determined in step (i) and step (ii) to construct a profile for said biomolecule;
(iv) comparing the profile obtained in step (iii) with at least one reference profile to identify the biomolecule.

2. The method of claim 1 wherein the first and second surfaces are different.

3. The method of claim 1 wherein the second surface is the same as the first surface but the sample is contacted with the second surface under at least one altered condition in comparison to the first surface.

4. The method of claim 3 wherein the altered condition is selected from any one of: temperature; pH; ionic strength, redox potential, electric potential applied between surface and solution and presence of a reagent; optionally wherein the reagent is selected from any one or more of: detergent, chaotropic agent, reducing agent, buffer, ion, salt, solvent, denaturing agent, and/or cross-linking agent.

5. A method as claimed in any preceding claim
wherein the mobility of at least one single biomolecule is tracked using interferometric light scattering apparatus; and/or wherein the mobility of said biomolecule is defined by:
(a) distance travelled by the biomolecule on the surface;
(b) speed of movement of the biomolecule on the surface;
(c) length of time the biomolecule interacts with the surface; and/or
(d) frequency of interaction of the biomolecule with the surface.

6. A method as claimed in any preceding claim wherein step (i) may be repeated one or more times with a further surface and the mobility of the biomolecule on this surface is used to construct the profile in step (iii); said further surface may be a different surface or may be the same surface as the first and/or second surface but the sample is contacted under at least one altered condition.

7. A method according to any preceding claim wherein any one of the surfaces may be selected from any one of the following: glass, diamond, plastic, polymeric material (e.g. cyclic olefin copolymer, polyvinyl, polyethylene (PE) including for example polyethylene terephthalate (PET) and high-density polyethylene (HDPE) and low-density polyethylene (LDPE), polyacrylate (acrylic), polystyrene (PS) including high impact polystyrene (HIPS), silicone, polyester (for example polylactic acid (PLA) or polylactic coglycolic acid (PGLA)), polyurethane, polypropylene (PP), polyamide (nylon), Acrylonitrile butadiene styrene (ABS), Polyethylene/Acrylonitrile Butadiene Styrene (PE/ABS), bakelite, rubber, latex, polycarbonate (PC), Polycarbonate/Acrylonitrile Butadiene Styrene (PC/ABS) and polyvinyl chloride including for example polyvinylidene chloride (PVDC),and sapphire.

8. A method according to any preceding claim wherein any one of the surfaces is activated, coated, treated and/or derivatised; optionally wherein the surface is activated, coated, treated and/or derivatised with any one or more of functionalised PEG, silanes, carboxy-silanes, amines, aminosilane, trimethylchlorosilane, aldehyde modification with amine modification reagents, epoxy modification, carboxylate modification, NHS, HOBt, TBTU, PAMAM; diazo, and supramolecules.

9. A method according to any one of claims 1 to 6 wherein any one of the surfaces is a lipid membrane, optionally a lipid bilayer.

10. A method as claimed in any preceding claim wherein at least one measurement from step (i) or step (ii) is used to determine the mass of the biomolecule.

11. A method according to any preceding claim wherein the surface and/or condition applied to the surface are selected to determine a physical characteristic of the biomolecule; optionally wherein the physical characteristic is selected from any one or more of: charge, pl, size, binding affinity, lipophilicity and/or hydrophobicity.

12. A method for release testing a biomolecule during manufacture, comprising the use of a method as defined in any one of claims 1 to 11.

13. A method as claimed in any preceding claim wherein the biomolecule is a protein, a carbohydrate, a lipid, a nucleic acid, a virus or virus like particle, a lipid nanoparticle or lipoplex.

14. A method as described in any preceding claim wherein the method can distinguish between different variants of a biomolecule; optionally wherein the variant is a serotype, an isoform, a glycoform, a lipoform, or a mutant.

15. A method according to claim 14 wherein the variants of the biomolecule have the same mass when measured by interferometric light scattering apparatus.

16. A method according to any preceding claim wherein a) the interferometric light scattering apparatus is a mass photometer; and/or b) wherein the measurements are taken at the interface between the surface and the sample; and/or c)wherein said biomolecule is not labelled; and/or d) wherein a fresh sample is used for each surface; and/or e) wherein the reference profile is prepared using a known biomolecule.

## Patentansprüche

1. Verfahren zur Identifizierung eines Biomoleküls in einer Probe unter Verwendung einer interferometrischen Lichtstreuvorrichtung; wobei das Verfahren Folgendes umfasst:
(i) Kontaktieren der Probe mit einer ersten Oberfläche und Durchführen einer Vielzahl von Messungen des Biomoleküls auf der ersten Oberfläche und Verwenden der Messungen, um die Mobilität des Biomoleküls auf der Oberfläche zu bestimmen;
(ii) Wiederholen von Schritt (i) unter Verwendung einer zweiten Oberfläche und Bestimmen der Mobilität des Biomoleküls auf der Oberfläche;
(iii) Verwenden der Mobilität des Biomoleküls, die in Schritt (i) und Schritt (ii) bestimmt wird, um ein Profil für das Biomolekül zu konstruieren;
(iv) Vergleichen des Profils, das in Schritt (iii) erhalten wird, mit zumindest einem Referenzprofil, um das Biomolekül zu identifizieren.

2. Verfahren nach Anspruch 1, wobei sich die erste und die zweite Oberfläche unterscheiden.

3. Verfahren nach Anspruch 1, wobei die zweite Oberfläche die gleiche wie die erste Oberfläche ist, aber die Probe mit der zweiten Oberfläche unter zumindest einem veränderten Zustand im Vergleich zu der ersten Oberfläche kontaktiert wird.

4. Verfahren nach Anspruch 3, wobei der veränderte Zustand ausgewählt ist aus einem beliebigen von: Temperatur; pH; Ionenstärke, Redoxpotential, elektrischem Potential, das zwischen Oberfläche und Lösung angelegt ist, und Vorhandensein eines Reagens; wobei optional das Reagens ausgewählt ist aus einem beliebigen oder mehreren von: Detergens, chaotropem Mittel, Reduktionsmittel, Puffer, Ion, Salz, Lösungsmittel, Denaturierungsmittel und/oder Vernetzungsmittel.

5. Verfahren nach einem vorhergehenden Anspruch,
wobei die Mobilität von zumindest einem einzelnen Biomolekül unter Verwendung einer interferometrischen Lichtstreuvorrichtung verfolgt wird; und/oder wobei die Mobilität des Biomoleküls definiert ist durch:
(a) Entfernung, die durch das Biomolekül auf der Oberfläche zurückgelegt wird;
(b) Bewegungsgeschwindigkeit des Biomoleküls auf der Oberfläche;
(c) Länge an Zeit, die das Biomolekül mit der Oberfläche interagiert; und/oder
(d) Häufigkeit der Interaktion des Biomoleküls mit der Oberfläche.

6. Verfahren nach einem vorhergehenden Anspruch, wobei Schritt (i) einmal oder mehrmals mit einer weiteren Oberfläche wiederholt werden kann und die Mobilität des Biomoleküls auf dieser Oberfläche verwendet wird, um das Profil in Schritt (iii) zu konstruieren; wobei die weitere Oberfläche eine andere Oberfläche sein kann oder dieselbe Oberfläche wie die erste und/oder zweite Oberfläche sein kann, aber die Probe unter zumindest einem veränderten Zustand kontaktiert wird.

7. Verfahren nach einem vorhergehenden Anspruch, wobei eine beliebige der Oberflächen aus einem beliebigen des Folgenden ausgewählt sein kann: Glas, Diamant, Kunststoff, Polymermaterial (z. B. cyclisches Olefin-Copolymer, Polyvinyl, Polyethylen (PE) beinhaltend zum Beispiel Polyethylenterephthalat (PET) und Polyethylen hoher Dichte (HDPE) und Polyethylen niedriger Dichte (LDPE), Polyacrylat (Acryl), Polystyrol (PS) beinhaltend hochschlagfestes Polystyrol (HIPS), Silikon, Polyester (zum Beispiel Polymilchsäure (PLA) oder Polymilch-Coglykolsäure (PGLA)), Polyurethan, Polypropylen (PP), Polyamid (Nylon), Acrylonitril-Butadien-Styrol (ABS), Polyethylen/Acrylonitril-Butadien-Styrol (PE/ABS), Bakelit, Kautschuk, Latex, Polycarbonat (PC), Polycarbonat/Acrylonitril-Butadien-Styrol (PC/ABS) und Polyvinylchlorid beinhaltend zum Beispiel Polyvinylidenchlorid (PVDC) und Saphir.

8. Verfahren nach einem vorhergehenden Anspruch, wobei eine beliebige der Oberflächen aktiviert, beschichtet, behandelt und/oder derivatisiert ist; wobei optional die Oberfläche mit einem oder mehreren von funktionalisiertem PEG, Silanen, Carboxysilanen, Aminen, Aminosilan, Trimethylchlorsilan, Aldehydmodifikation mit Aminmodifikationsreagenzien, Epoxymodifikation, Carboxylatmodifikation, NHS, HOBt, TBTU, PAMAM; Diazo und Supramolekülen aktiviert, beschichtet, behandelt und/oder derivatisiert ist.

9. Verfahren nach einem der Ansprüche 1 bis 6, wobei eine beliebige der Oberflächen eine Lipidmembran, optional eine Lipiddoppelschicht ist.

10. Verfahren nach einem vorhergehenden Anspruch, wobei zumindest eine Messung aus Schritt (i) oder Schritt (ii) verwendet wird, um die Masse des Biomoleküls zu bestimmen.

11. Verfahren nach einem vorhergehenden Anspruch, wobei die Oberfläche und/oder der Zustand angelegt an die Oberfläche ausgewählt sind, um eine physikalische Eigenschaft des Biomoleküls zu bestimmen; wobei optional die physikalische Eigenschaft ausgewählt ist aus einem beliebigen oder mehreren von: Ladung, pI, Größe, Bindungsaffinität, Lipophilie und/oder Hydrophobie.

12. Verfahren zum Freigabetesten eines Biomoleküls während Herstellung, umfassend die Verwendung eines Verfahrens wie in einem der Ansprüche 1 bis 11 definiert.

13. Verfahren nach einem vorhergehenden Anspruch, wobei das Biomolekül ein Protein, ein Kohlenhydrat, ein Lipid, eine Nukleinsäure, ein Virus oder virusähnliches Partikel, ein Lipidnanopartikel oder Lipoplex ist.

14. Verfahren wie in einem vorhergehenden Anspruch beschrieben, wobei das Verfahren zwischen verschiedenen Varianten eines Biomoleküls unterscheiden kann; wobei optional die Variante ein Serotyp, eine Isoform, eine Glycoform, eine Lipoform oder eine Mutante ist.

15. Verfahren nach Anspruch 14, wobei die Varianten des Biomoleküls die gleiche Masse aufweisen, wenn durch eine interferometrische Lichtstreuvorrichtung gemessen.

16. Verfahren nach einem vorhergehenden Anspruch, wobei a) die interferometrische Lichtstreuvorrichtung ein Massenphotometer ist; und/oder b) wobei die Messungen an der Schnittstelle zwischen der Oberfläche und der Probe durchgeführt werden; und/oder c) wobei das Biomolekül nicht markiert ist; und/oder d) wobei eine frische Probe für jede Oberfläche verwendet wird; und/oder e) wobei das Referenzprofil unter Verwendung eines bekannten Biomoleküls zubereitet wird.

## Revendications

1. Procédé d'identification d'une biomolécule dans un échantillon à l'aide d'un appareil de diffusion de lumière interférométrique ; ledit procédé comprenant :
(i) la mise en contact dudit échantillon avec une première surface et la prise d'une pluralité de mesures de ladite biomolécule sur ladite première surface et l'utilisation des mesures pour déterminer la mobilité de ladite biomolécule sur ladite surface ;
(ii) la répétition de l'étape (i) en utilisant une seconde surface et la détermination de la mobilité de ladite biomolécule sur ladite surface ;
(iii) l'utilisation de la mobilité de la biomolécule déterminée à l'étape (i) et à l'étape (ii) pour construire un profil pour ladite biomolécule ;
(iv) la comparaison du profil obtenu à l'étape (iii) avec au moins un profil de référence pour identifier la biomolécule.

2. Procédé de la revendication 1, dans lequel les première et seconde surfaces sont différentes.

3. Procédé de la revendication 1, dans lequel la seconde surface est la même que la première surface, mais l'échantillon est mis en contact avec la seconde surface dans au moins une condition modifiée par rapport à la première surface.

4. Procédé de la revendication 3, dans lequel la condition modifiée est choisie parmi l'un quelconque parmi : la température ; le pH ; la force ionique, le potentiel redox, le potentiel électrique appliqué entre une surface et une solution et la présence d'un réactif ; éventuellement dans lequel le réactif est choisi parmi l'un quelconque ou plusieurs parmi : un détergent, un agent chaotropique, un agent réducteur, un tampon, un ion, un sel, un solvant, un agent dénaturant et/ou un agent de réticulation.

5. Procédé selon une quelconque revendication précédente, dans lequel la mobilité d'au moins une biomolécule unique est suivie à l'aide d'un appareil de diffusion de lumière interférométrique ; et/ou dans lequel la mobilité de ladite biomolécule est définie par :
(a) une distance parcourue par la biomolécule sur la surface ;
(b) une vitesse de déplacement de la biomolécule sur la surface ;
(c) une durée pendant laquelle la biomolécule interagit avec la surface ; et/ou
(d) une fréquence d'interaction de la biomolécule avec la surface.

6. Procédé selon une quelconque revendication précédente, dans lequel l'étape (i) peut être répétée une ou plusieurs fois avec une surface supplémentaire et la mobilité de la biomolécule sur cette surface est utilisée pour construire le profil à l'étape (iii) ; ladite surface supplémentaire peut être une surface différente ou peut être la même surface que la première et/ou la seconde surface, mais l'échantillon est mis en contact dans au moins une condition modifiée.

7. Procédé selon une quelconque revendication précédente, dans lequel l'une quelconque des surfaces peut être choisie parmi l'un quelconque parmi : le verre, le diamant, le plastique, un matériau polymère (par exemple un copolymère d'oléfine cyclique, le polyvinyle, le polyéthylène (PE), y compris par exemple le polyéthylène téréphtalate (PET) et le polyéthylène haute densité (HDPE) et le polyéthylène basse densité (LDPE), le polyacrylate (acrylique), le polystyrène (PS), y compris le polystyrène à haute résistance aux chocs (HIPS), le silicone, le polyester (par exemple l'acide polylactique (PLA) ou l'acide polylactique-co-glycolique (PGLA)), le polyuréthane, le polypropylène (PP), le polyamide (nylon), l'acrylonitrile butadiène styrène (ABS), le polyéthylène/acrylonitrile butadiène styrène (PE/ABS), la bakélite, le caoutchouc, le latex, le polycarbonate (PC), le polycarbonate/acrylonitrile butadiène styrène (PC/ABS) et le polychlorure de vinyle, y compris par exemple le polychlorure de vinylidène (PVDC), et le saphir.

8. Procédé selon une quelconque revendication précédente, dans lequel l'une quelconque des surfaces est activée, revêtue, traitée et/ou dérivatisée ; éventuellement dans lequel la surface est activée, revêtue, traitée et/ou dérivatisée avec l'un quelconque ou plusieurs parmi le PEG fonctionnalisé, des silanes, des carboxy-silanes, des amines, l'aminosilane, le triméthylchlorosilane, une modification d'aldéhyde avec des réactifs de modification d'amine, une modification d'époxy, une modification de carboxylate, le NHS, le HOBt, le TBTU, la PAMAM ; un diazo et des supramolécules.

9. Procédé selon l'une quelconque des revendications 1 à 6, dans lequel l'une quelconque des surfaces est une membrane lipidique, éventuellement une bicouche lipidique.

10. Procédé selon une quelconque revendication précédente, dans lequel au moins une mesure de l'étape (i) ou de l'étape (ii) est utilisée pour déterminer la masse de la biomolécule.

11. Procédé selon une quelconque revendication précédente, dans lequel la surface et/ou la condition appliquée(s) à la surface est/sont choisie(s) pour déterminer une caractéristique physique de la biomolécule ; éventuellement dans lequel la caractéristique physique est choisie parmi l'un quelconque ou plusieurs parmi : la charge, le pl, la taille, l'affinité de liaison, la lipophilie et/ou l'hydrophobicité.

12. Procédé permettant de tester la libération d'une biomolécule pendant la fabrication, comprenant l'utilisation d'un procédé selon l'une quelconque des revendications 1 à 11.

13. Procédé selon une quelconque revendication précédente, dans lequel la biomolécule est une protéine, un glucide, un lipide, un acide nucléique, un virus ou une particule de type virus, une nanoparticule lipidique ou un lipoplexe.

14. Procédé selon une quelconque revendication précédente, dans lequel le procédé peut faire la distinction entre différents variants d'une biomolécule ; éventuellement dans lequel le variant est un sérotype, une isoforme, une glycoforme, une lipoforme ou un mutant.

15. Procédé selon la revendication 14, dans lequel les variants de la biomolécule comportent la même masse lorsqu'ils sont mesurés par un appareil de diffusion de lumière interférométrique.

16. Procédé selon une quelconque revendication précédente, dans lequel a) l'appareil de diffusion de lumière interférométrique est un photomètre de masse ; et/ou b) dans lequel les mesures sont prises au niveau de l'interface entre la surface et l'échantillon ; et/ou c) dans lequel ladite biomolécule n'est pas marquée ; et/ou d) dans lequel un nouvel échantillon est utilisé pour chaque surface ; et/ou e) dans lequel le profil de référence est préparé en utilisant une biomolécule connue.
